Europäisches Patentamt

European Patent Office

Office européen des brevets

(11) Numéro de publication: **0 051 524**
**B1**

(12) FASCICULE DE BREVET EUROPEEN

(45) Date de publication du fascicule du brevet: **12.12.84**

(51) Int. Cl.³: **C 07 C 17/02,** C 07 C 19/045, C 07 C 17/156

(21) Numéro de dépôt: **81401676.2**

(22) Date de dépôt: **23.10.81**

(54) Procédé de traitement des gaz résiduaires inertes contenant de l'éthylène.

(30) Priorité: **04.11.80 FR 8023510**

(43) Date de publication de la demande:
**12.05.82 Bulletin 82/19**

(45) Mention de la délivrance du brevet:
**12.12.84 Bulletin 84/50**

(84) Etats contractants désignés:
**DE FR NL**

(56) Documents cités:
**DE-A-2 733 502**
**FR-A- 491 792**
**FR-A-1 575 262**

(73) Titulaire: **ATOCHEM**
**12/16, allée des Vosges**
**F-92400 Courbevoie (FR)**

(72) Inventeur: **Gorny, Bernard Simon Jean Marie**
**Le Thicaud**
**Herbeys F-38320 Eybens (FR)**
Inventeur: **Mathais, Henri**
**Hameau de Saint-Didier Chemin de l'Indiennerie**
**F-69370 Saint-Didier au Mont d'Or (FR)**

(74) Mandataire: **Rochet, Michel et al**
**ATOCHEM Département Propriété Industrielle**
**Cédex 22**
**F-92091 Paris la Défense (FR)**

Courier Press, Leamington Spa, England.

## Description

L'invention concerne une traitement des gaz résiduaires inertes contenant de l'éthylène provenant de la fabrication du dichloréthane par oxychloration de l'éthylène.

Les procédés industriels de fabrication du dichloréthane par réaction de l'oxygène sur un mélange d'acide chlorhydrique et d'éthylène ne conduisent jamais à une réaction totale et les gaz issus du réacteur d'oxychloration contiennent toujours de l'éthylène non transformé. Cette quantité d'éthylène représente une perte non négligeable et plusieurs procédés ont été développés pour réduire cette perte en transformant l'éthylène résiduaire en dichloréthane par une chloration complémentaire.

Dans le brevet français 1 421 903, le gaz issu de l'oxychloration, après refroidissement pour condenser le dichloréthane formé, est traité en phase vapeur par du chlore sur un catalyseur à base de fer. Ce traitement se fait à température élevée — de 80 à 250°C — ce qui est un inconvénient car les mélanges gazeux contenant du chlore humide sont très corrosifs, ce qui implique soit l'utilisation de matériau coûteux, soit une augmentation des frais d'entretien. En outre, il faut réchauffer le flux gazeux à une température suffisante pour que la réaction puisse se faire assez rapidement, ce qui entraîne une consommation d'énergie importante. Les mêmes critiques peuvent être faites au brevet français 1 575 262 et à la demande allemande 2 733 502 qui dans des conditions très voisines décrivent l'utilisation de l'alumine comme catalyseur.

Pour éviter ces inconvénients il a été proposé d'effectuer la chloration en phase liquide dans un solvant non réactif. C'est ainsi que le brevet britannique 1 364 610 décrit un procédé dans lequel les gaz résiduaires d'oxychloration passent après séchage dans un réacteur parcouru par un flux du liquide inerte, de préférence du 1,2-dichloréthane, en même temps que du chlore est introduit en quantité suffisante pour transformer une grande partie de l'éthylène résiduaire. La réaction de chloration a lieu entre 35 et 85°C en présence de chlorure ferrique. Cependant à cause de la grande dilution de l'éthylène dans le gaz résiduaire, cette technique nécessite la mise en contact d'un très fort débit de dichloréthane, ce qui conduit à des réacteurs de dimensions très importantes, donc à un investissement coûteux par rapport à la production de dichloréthane attendue.

Le brevet français 491.792 cite un procédé de fabrication de dichloréthane par réaction de l'éthylène sur le chlore à au moins 90°C en présence de charbon de bois ou de noir animal, sans donner d'autres précisions sur les conditions de réaction. Compte tenu que selon PASCAL, Vol. VIII, pp. 768—769, le chlore réagit sur le CO en présence de charbon actif pour donner du phosgène, la technique signalée par ce brevet français ne peut être envisageable à priori pour le traitement des gaz résiduaires provenant de la fabrication du dichloréthane par oxychloration de l'éthylène, gaz résiduaires qui par définition contiennent du CO.

La présente invention obvie à ces inconvénients. Elle permet de transformer en 1,2-dichlorèthane, avec un excellent rendement, l'éthylène contenu dans les gaz résiduaires d'oxychloration, et opère à une température assez basse qui limite les dépenses d'énergie dues au réchauffage des gaz et supprime les risques de corrosion, sans faire appel à la circulation d'un fort débit de solvant.

Dans ce but, le gaz résiduaire contenant l'éthylène est additionné de chlore dans un rapport molaire $Cl_2/C_2H_4$ généralement compris entre 0,5 et 1,5 et plus précisément entre 0,8 et 1,1. Le mélange ainsi obtenu est envoyé sur du charbon actif comme catalyseur avec un temps de contact compris entre 0,1 et 10 secondes et de préférence entre 0,2 et 5 secondes (le temps de contact étant défini comme le rapport entre le volume de catalyseur (ml) et le débit volumique (ml/s)).

La température de la réaction est comprise entre 15 et 80°C.

Le taux de transformation de l'éthylène est compris entre 90 et 100 %, le rendement en dichloréthane étant pratiquement quantitatif.

Le présent procédé concerne les gaz résiduaires d'oxychloration de l'éthylène, mais tout gaz résiduaire inerte contenant de l'éthylène ou un autre hydrocarbure insaturé peut être traité de la même manière, permettant ainsi une récupération plus facile d'un produit de poids moléculaire plus élevé.

Les exemples suivants illustrent l'invention

### Exemple 1

On traite 191 l/h d'un effluent gazeux d'oxychloration de l'éthylène dont la composition est la suivante:

| | |
|---|---|
| $CO_2$ | 2,1 % |
| CO | 1,2 % |
| $C_2H_4$ | 1,2 % |
| HCL | 0,1 % |
| $CH_2Cl—CH_2Cl$ | 0,03 % |
| $O_2$ | 6,0 % |
| $N_2$ | 89,37 % |

par 2,23 l/h de chlore en phase vapeur (soit un rapport molaire $Cl_2/C_2H_4$ de 0,973) à une température de 70°C sur 175 ml de charbon actif (Acticarbone AC 40 de la Société CESA S.A.). Le temps de contact est donc de 2,6 secondes.

Dans ces conditions, le taux de transformation de l'éthylène est de 97,5 %.

### Exemple 2

On traite 600 l/h de l'effluent gazeux de l'exemple 1 par 6,9 l/h de chlore (rapport molaire $Cl_2/C_2H_4 = 0,958$), à une température de 70°C, sur 88 ml de charbon actif, ce qui représente un temps de contact de 0,4 seconde.

Le taux de transformation de l'éthylène est de 96 %.

### Exemple 3

On traite 600 l/h de l'effluent gazeux de l'exemple 1 par 7,9 l/h de chlore (rapport molaire $Cl_2/C_2H_4$ = 1,097) dans les mêmes conditions que dans l'exemple 2, à savoir à une température de 70°C sur 88 ml de charbon actif (temps de contact: 0,4 seconde).

Le taux de transformation de l'éthylène est de 96 % comme dans l'exemple 2. En revanche, on retrouve, dans le gaz après traitement, l'excès de chlore qui n'a pas réagi, c'est-à-dire 1,02 l/h, mais celui-ci peut être éliminé facilement par des techniques classiques.

### Exemple 4

On traite 180 l/h d'un effluent gazeux d'oxychloration de l'éthylène dont la composition est la suivante:

| | |
|---|---|
| $CO_2$ | 2,5 % |
| CO | 1,0 % |
| $C_2H_4$ | 1,27 % |
| $CH_2$—Cl—$CH_2Cl$ | 0,12 % |
| $O_2$ | 6,0 % |
| $N_2$ | 89,11 % |

par 2,35 l/h de chlore (rapport molaire $Cl_2/C_2H_4$ = 0,984) à une température de 20°C sur 175 ml de charbon actif. Le temps de contact est de 3,5 secondes.

Dans ces conditions, le taux de transformation de l'éthylène est de 97,5 %.

### Exemple 5

On traite 216 l/h d'un effluent gazeux qui a la composition suivante:

| | |
|---|---|
| $CO_2$ | 1,9 % |
| CO | 0,8 % |
| $C_2H_4$ | 0,7 % |
| $O_2$ | 6,0 % |
| $N_2$ | 90,6 % |

par 1,38 l/h de chlore en phase vapeur, à une température de 50°C, sur 175 ml de charbon actif, c'est-à-dire avec un temps de contact de 2,9 secondes.

Dans ces conditions, où le rapport molaire chlore/éthylène n'est que de 0,913, le taux de transformation de l'éthylène est de 89 %.

## Revendications

1. Procédé de traitement des gaz résiduaires, contenant de l'éthylène et du CO, provenant de la fabrication du dichloréthane par oxychloration de l'éthylène par chloration en phase vapeur du gaz résiduaire avec du chlore en présence de catalyseur caractérisé en ce que la réaction s'effectue à une température comprise entre 15 et 80°C en présence de charbon actif comme catalyseur, le temps de contact avec ce dernier étant compris entre 0,1 et 10 secondes.

2. Procédé selon la revendication 1 caractérisé en ce que le rapport molaire chlore éthylène est compris entre 0,5 et 1,5 et plus précisément entre 0,8 et 1,1.

## Patentansprüche

1. Verfahren zur Behandlung von Ethylen und CO enthaltenden Restgasen, die bei der Herstellung von Dichlorethan durch Oxychlorierung von Ethylen anfallen, durch Chlorieren des Restgases in der Gasphase mit Chlor in Gegenwart eines Katalysators, dadurch gekennzeichnet, daß die Reaktion bei einer Temperatur zwischen 15 und 80°C in Gegenwart von Aktivkohle als Katalysator abläuft und die Kontaktzeit mit dem Katalysator zwischen 0,1 und 10 sec liegt.

2. Verfahren nach Anspruch 1, dadurch gekennzeichnet, daß das Molverhältnis zwischen Chlor und Ethylen zwischen 0,5 und 1,5, insbesondere zwischen 0,8 und 1,1 liegt.

## Claims

1. Process for the treatment of residual gases, containing ethylene and CO, originating from the manufacture of dichloroethane by oxychlorination of ethylene, by vapour phase chlorination of the residual gas with chlorine in the presence of a catalyst, characterised in that the reaction is carried out at a temperature of between 15 and 80°C in the presence of active charcoal as the catalyst, the contact time with the latter being between 0.1 and 10 seconds.

2. Process according to Claim 1, characterised in that the molar ratio of chlorine to unsaturated ethylene is between 0.5 and 1.5 and more precisely between 0.8 and 1.1.